# FASCICULE DE BREVET EUROPEEN

(11) **EP 0 793 482 B1**
(45) Date de publication et mention de la délivrance du brevet: **14.08.2002**
(21) Numéro de dépôt: 96931850.0
(22) Date de dépôt: 16.09.1996
(51) Int. Cl.: A61K 7/48, A61K 7/06

(54) **COMPOSITION POUR LE TRAITEMENT DES MATIERES KERATINIQUES COMPRENANT AU MOINS UN POLYMERE SILICONE GREFFE A SQUELETTE POLYSILOXANIQUE GREFFE PAR DES MONOMERES ORGANIQUES NON-SILICONES ET AU MOINS UN POLYMERE ANIONIQUE**
ZUSAMMENSETZUNG ZUR BEHANDLUNG VON KEATINSUBSTANZEN, DIE MINDESTENS EIN GEPFROPFTES SILICONPOLYMER MIT POLYSILOXANGERÜST, AUF DAS NICHT SILICONHALTIGE, ORGANISCHE MONOMERE GEPFROPFT SIND, UND MINDESTENS EIN ANIONISCHES POLYMER ENTHÄLT
COMPOSITION FOR TREATING KERATINOUS MATERIAL, INCLUDING AT LEAST ONE SILICONE-GRAFTED POLYMER WITH A POLYSILOXANE BACKBONE GRAFTED BY NON-SILICONE ORGANIC MONOMERS AND AT LEAST ONE ANIONIC POLYMER

(30) Priorité: 29.09.1995 FR 9511478
(43) Date de publication de la demande: 10.09.1997
(73) Titulaire: L'OREAL, 75008 Paris (FR)
(72) Inventeur: DUBIEF, Claude, F-78150 Le Chesnay (FR); DUPUIS, Christine, F-75018 Paris (FR); CAUWET-MARTIN, Danièle, F-75011 Paris (FR)
(74) Mandataire: Miszputen, Laurent
(86) Numéro de dépôt international: FR9601432
(87) Numéro de publication internationale: WO9712592

(56) Documents cités:
- EP-A- 0 370 764
- EP-A- 0 388 582
- EP-A- 0 437 075
- EP-A- 0 463 780
- EP-A- 0 582 152
- EP-A- 0 640 643
- EP-A- 0 756 859
- WO-A-93/23009
- WO-A-95/03776
- WO-A-95/23581
- WO-A-97/01321
- US-A- 4 983 418
- US-A- 5 034 218

## Description

La présente invention a trait à une composition cosmétique ou dermatologique pour le traitement des matières kératiniques, en particulier des cheveux comprenant au moins un polymère siliconé greffé, à squelette polysiloxanique greffé par des monomères organiques non-siliconés et au moins un polymère anionique.

On connaît dans l'état de la technique des polymères siliconé greffé tels que ceux décrits dans les demandes de brevet EP-A-0582152 et WO 93/23009. Ces polymères sont proposés dans les compositions capillaires pour leurs propriétés coiffantes. Cependant, lorsque l'on utilise ces polymères, la tenue de la coiffure et le toucher des cheveux ne sont pas satisfaisants.

Le document WO97/01321 décrit une composition cosmétique sans transfert comprenant un polymère adhésif, un solvant volatile, une huile non volatile, des particules solides et une cire. Les exemples 4 et 5 concernent des compositions pour les lèvres comprenant du VS70-5 et du SA70-5, lesquels sont tous deux des polymères non ioniques.

Le document EP 756 859 concerne des compositions capillaires comprenant un copolymérisat quaternaire aminoalkyldiméthylpolysiloxane / polyéthyloxazoline, dont le procédé de préparation est indiqué dans EP 640 643. Ces polymères siliconés sont différents de ceux mis en oeuvre dans la présente invention.

La demanderesse a découvert de façon surprenante qu'en associant au moins un polymère siliconé greffé avec au moins un polymère anionique dans un rapport en poids polymère anionique /polymère siliconé greffé compris entre 0,25 et 15, on obtenait des propriétés de toucher et de douceur des cheveux sensiblement supérieures à celles obtenues avec chaque polymère utilisé seul.

La composition selon l'invention est donc essentiellement caractérisée par le fait qu'elle comprend dans un milieu cosmétiquement ou dermatologiquement acceptable au moins un polymère siliconé greffé, à squelette polysiloxanique greffé par des monomères organiques non-siliconés et au moins un polymère anionique dans un rapport en poids polymère anionique / polymère siliconé greffé compris entre 0,25 et 15.

Dans ce qui suit, on entend désigner par polymère siliconé, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les polymères siliconés qui doivent être utilisés sont ceux qui comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone. Ce polymère est défini à la revendication 1.

Ces polymères siliconés peuvent être des produits commerciaux existants, ou encore être obtenus selon tout moyen connu de l'homme de l'art, en particulier par réaction entre (i) une silicone de départ correctement fonctionnalisée sur un ou plusieurs de ces atomes de silicium et (ii) un composé organique non-siliconé lui-même correctement fonctionnalisé par une fonction qui est capable de venir réagir avec le ou les groupements fonctionnels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionnels -SH avec ces mêmes groupements vinyliques.

Des exemples de polymères siliconés convenant à la mise en oeuvre de la présente invention, ainsi que leur mode particulier de préparation, sont notamment décrits dans les demandes de brevets EP-A-0 582 152, WO 93/23009 et WO 95/03776.

Selon un mode particulièrement préféré de réalisation de la présente invention, le polymère siliconé mis en oeuvre comprend le résultat de la copolymérisation radicalaire entre d'une part au moins un monomère organique anionique non-siliconé présentant une insaturation éthylénique et/ou un monomère organique hydrophobe non-siliconé présentant une insaturation éthylénique et d'autre part une silicone présentant dans sa chaîne au moins un groupement fonctionnel capable de venir réagir sur lesdites insaturations éthyléniques desdits monomères non-siliconés en formant une liaison covalente, en particulier des groupements thio-fonctionnels.

Selon la présente invention, lesdits monomères anioniques à insaturation éthylénique sont de préférence choisis, seuls ou en mélange, parmi les acides carboxyliques insaturés, linéaires ou ramifiés, éventuellement partiellement ou totalement neutralisés sous la forme d'un sel, ce ou ces acides carboxyliques insaturés pouvant être plus particulièrement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le polymère siliconé greffé final, le groupement organique à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Selon la présente invention, les monomères hydrophobes à insaturation éthylénique sont de préférence choisis, seuls ou en mélange, parmi les esters d'acide acrylique alcanols et/ou les esters d'acide méthacrylique d'alcanols. Les alcanols sont de préférence en C₁-C₁₈ et plus particulièrement en C₁-C₁₂. Les monomères préférentiels sont choisis dans le groupe constitué par le (méth)acrylate d'isooctyle, le 2-éthylhexyl(méth)acrylate, le (méth)acrylate de lauryle, le (méth) acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle ou leurs mélanges.

La famille de polymères siliconés greffés nécessaire à la mise en oeuvre de la présente invention est constituée par les polymères siliconés comportant dans leur structure le motif de formule (I) suivant : 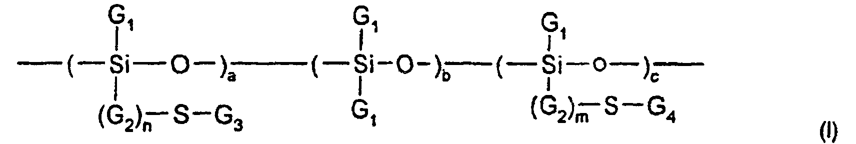 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo) polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (I) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence du type (méth)acrylate d'isobutyle ou de méthyle.

Des exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate de méthyle.

D'autres exemples de polymères siliconés répondant à la formule (I) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés de l'invention varie de 10 000 à 1 000 000 environ, et encore plus préférentiellement de 10 000 à 100 000 environ.

Le polymère siliconé greffé est utilisé de préférence en une quantité allant de 0,01 à 15% en poids du poids total de la composition. Encore plus préférentiellement, cette quantité varie de 0,1 à 10% en poids.

Selon l'invention, on peut utiliser tout polymère anionique connu en soi. Ces polymères sont des polymères fixants, c'est à dire ayant pour fonction de fixer temporairement la forme de la coiffure.
Bien entendu, on peut utiliser un ou plusieurs polymères anioniques.

Ainsi, les polymères anioniques généralement utilisés sont des polymères comportant des groupements dérivés d'acide carboxylique, sulfonique ou phosphorique et ont un poids moléculaire compris entre environ 500 et 5.000.000.

Les groupements carboxyliques sont apportés par des monomères mono ou diacides carboxyliques insaturés tels que ceux répondant à la formule : 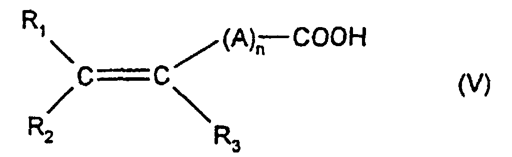 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur ou carboxyle, R₃ désigne un atome d'hydrogène, un groupement alkyle inférieur, un groupement -CH₂-COOH, phényle ou benzyle.

Dans la formule précitée un radical alkyle inférieur désigne de préférence un groupement ayant 1 à 4 atomes de carbone et en particulier, méthyle et éthyle.

Les polymères anioniques à groupements carboxyliques préférés selon l'invention sont:
A) les homo- ou copolymères d'acide acrylique ou méthacrylique ou leurs sels et en particulier les produits vendus sous les dénominations VERSICOL E ou K par la société ALLIED COLLOID, ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et le copolymère d'acide méthacrylique et d'acrylate d'éthyle vendu sous la dénomination LUVIMER MAEX par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées. Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi:
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG par la société BASF, les copolymères dérivés d'acide crotonique tels que les terpolymères acétate de vinyle/tertio-butyl benzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX par la société BASF et le copolymère acétate de vinyle/acide crotonique vendu sous la dénomination LUVISET CA 66 par la société BASF et le terpolymère acétate de vinyle/acide crotonique/polyéthylèneglycol sous la dénomination ARISTOFLEX A par la société BASF.

Les polymères anioniques les plus particulièrement préférés sont choisis parmi le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX par la société BASF, le terpolymère de vinylpyrrolidone/acide acrylique/méthacrylate de lauryle vendu sous la dénomination ACRYLIDONE LM par la société ISP.

Selon l'invention, on peut également utiliser les polymères anioniques sous forme de latex ou de pseudolatex, c'est à dire sous forme d'une dispersion aqueuse de particules de polymères insolubles.

Le rapport en poids polymère anionique/polymère siliconé greffé est de préférence compris entre 0,3 et 8

Selon l'invention, le ou les polymères anioniques peuvent représenter de 0,1 % à 20 % en poids, de préférence de 0,2 % à 15 % en poids, et encore plus préférentiellement de 0,5 % à 10 % en poids, du poids total de la composition finale.

Le milieu cosmétiquement ou dermatologiquement acceptable est de préférence constitué par de l'eau ou un mélange d'eau et de solvants cosmétiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, les éthers de glycol, les alkyléthers de glycol ou de diéthylèneglycol.

Les polymères siliconés greffés selon l'invention peuvent être dissous dans ledit milieu cosmétiquement acceptable ou utilisés sous forme de dispersion aqueuse de particules.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 20 % en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions selon l'invention sont utilisées comme produits rincés ou comme produits non-rincés notamment pour le lavage, le soin, le conditionnement, le maintien de la coiffure ou la mise en forme des matières kératiniques telles que les cheveux.

Elles sont plus particulièrement des produits de coiffage tels que des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Les compositions peuvent être également des shampooings, des compositions à rincer ou non, à appliquer avant ou après un shampooing, une coloration, une décoloration, une permanente ou un défrisage.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther. l'azote, l'air comprimé et leurs mélanges.

L'invention a encore pour objet un procédé de traitement des matières kératiniques telles que les cheveux consistant à appliquer sur ceux-ci une composition telle que définie précédemment puis à effectuer éventuellement un rinçage à l'eau.

L'invention va être maintenant plus complètement illustrée à l'aide des exemples suivants qui ne sauraient être considérés comme la limitant aux modes de réalisation décrits. (Dans ce qui suit MA signifie Matière Active).

### EXEMPLE 1 Spray de coiffage en flacon pompe

- Polymère siliconé greffé de formule (I) de structure polyméthyl/méthylsiloxane à groupements propyl thio-3 acide polyméthacrylate de méthyle 5 g
- Terpolymère acide acrylique / acrylate d'éthyle / N-tertiobutyl acrylamide ( ULTRAHOLD STRONG de BASF) 1 g
- Aminométhylpropanol neutralisation à 100% des deux polymères qsp
- Ethanol qsp 100 g

### EXEMPLE 2 Shampooing

- Polymère siliconé greffé de formule (I) de structure polyméthyl/méthylsiloxane à groupements propyl thio-3 acide polyméthacrylique et groupements propyl thio-3 1 g
- Lauryl (C₁₂/C₁₄ 70/30) éther sulfate de sodium oxyéthyléné à 2,2 moles d'oxyde d'éthylène en solution aqueuse à 28 % de MA vendu sous le nom d'Empicol ESB 3/FL par la société Albright et Wilson 10 g MA
- Cocoyibétaïne en solution aqueuse à 28 % de MA 4 g MA
- Terpolymère acétate de vinyle/acide crotonique/néodécanoate de vinyle vendu sous le nom de Résine 28-29-30 par National 1 g MA Starch (neutralisée par NaOH).
- Parfum, séquestrant, conservateur
- Eau q.s.p. 100 g

Le pH est ajusté à 7,5 par l'addition de soude.

### EXEMPLE 3 Spray de coiffage en flacon pompe

- Polymère siliconé greffé de formule (I) de structure polyméthyl/méthylsiloxane à groupements propyl thio-3 acide polyméthacrylique et groupements propyl thio-3 polyméthacrylate de méthyle 0,5 g
- Terpolymère acide méthacrylique / acrylate d'éthyle / acrylate de-tertiobutyle ( LUVIMER 100 P de BASF) 5 g
- Aminométhylpropanol neutralisation à 100% des deux polymères qsp
- Eau qsp 100 g

### EXEMPLE 4 Spray de coiffage en flacon pompe

- Polymère siliconé greffé de formule (I) de structure polyméthyl/méthylsiloxane à groupements propyl thio-3 acide polyméthacrylique et groupements propyl thio-3 polyméthacrylate de méthyle 2 g
- Copolymère acide méthacrylique / acide acrylique / acrylate d'éthyle / méthacrylate de méthyle en dispersion aqueuse à 25 % ( AMERHOLD DR 25 de AMERCHOL) 7 g MA
- Eau qsp 100 g

### EXEMPLE 5 Spray de coiffage en aérosol

- Polymère siliconé greffé de formule (I) de structure polyméthyl/méthylsiloxane à groupements propyl thio-3 acide polyméthacrylique et groupements propyl thio-3 polyméthacrylate de méthyle 5 g
- Copolymère acétate de vinyle/acide crotonique/tertlo-butyl-4-benzoate de vinyle (65/10/25) tel que décrit et préparé dans le brevet FR 2.697.160 2,5 g
- Aminométhylpropanol neutralisation à 100% du polymère siliconé greffé et du copolymère non siliconé qsp
- Ethanol qsp 100 g

### Schéma de pressurisation :

- Composition ci-dessus : 80 g
- Mélange ternaire de n-butane, isobutane et propane (23/55/22), vendu sous la dénomination "AEROGAZ 3,2 N par la société ELF AQUITAINE 5 g
- 1,1-difluoroéthane (SOLKANE 152 A de SOLVAY) 15 g

### EXEMPLE 6 Spray de coiffage en aérosol

- Polymère siliconé greffé de formule (I) de structure polyméthyl/méthylsiloxane à groupements propyl thio-3 acide polyméthacrylique et groupements propyl thio-3 polyméthacrylate de méthyle 4,6 g
- Copolymère acétate de vinyle/acide crotonique/tertio-butyl-4-benzoate de vinyle (65/10/25) tel que décrit et préparé dans le brevet FR 2.697.160 3 g
- Aminométhylpropanol neutralisation à 100% du polymère siliconé greffé et du copolymère non siliconé qsp
- Ethanol 38,5 g
- Eau qsp 100 g

### Schéma de pressurisation :

- Composition ci-dessus : 65g
- Diméthyléther 35 g

### EXEMPLE 7

On a préparé deux compositions selon l'invention A et B et on les a comparé aux compositions C et D contenant chacune un seul des deux polymères et à la composition E qui contient les deux polymères mais dans un rapport non compris dans l'invention. Les cinq compositions ont été conditionnées dans un flacon-pompe. Un panel de 5 testeurs expérimentés a évalué la douceur des cheveux après traitement avec ces compositions.

La notation allait de 0 (mauvais) à 5 (excellent), c'est à dire allant de pas du tout doux à extrêmement doux.

Les résultats sont rassemblés dans le tableau ci-après :

| **En gMA** | **A** **(Invention)** | **B** **(Invention)** | **C** **(Comparatif)** | **D** **(Comparatif)** | **E** **(Comparatif)** |
|---|---|---|---|---|---|
| **PSV**^{**(1)**} | 0,5 | 1 | 1,5 | ― | 1,3 |
| **GANTREZ ES 425**^{**(2)**} | 1 | 0,5 | ― | 1,5 | 0,2 |
| **AMP**^{**(3)**} neutralisation à 100% du ou des polymères | qs | qs | qs | qs | qs |
| **Eau qsp** | 100 | 100 | 100 | 100 | 100 |
| **R** | 2 | 0,5 | - | - | 0,15 |
| **Douceur** | 4 | 4,25 | 3,5 | 3,5 | 3,25 |
| R = GANTREZ ES 425 / PSV | | | | | |

| | | | | | |
|---|---|---|---|---|---|
| ⁽¹⁾PSV: Polymère siliconé greffé de formule (I) de structure polyméthyl/méthylsiloxane à groupements propyl thio-3 acide polyméthacrylique et groupements propyl thio-3 polyméthacrylate de méthyle. | | | | | |
| ⁽²⁾GANTREZ ES 425 : Copolymère méthylvinyléther/anhydride maléique mono estérifié vendu par la société ISP | | | | | |
| ⁽³⁾AMP: Amino-2 méthyl-2 propanol | | | | | |

Les cheveux traités avec les compositions A et B selon l'invention sont plus doux que ceux traités avec les compositions ne contenant qu'un seul des deux polymères. Lorsque les deux polymères sont présents dans un rapport qui n'est pas compris entre 0,25 et 15 (Composition E), il n'y a pas d'amélioration de la douceur des cheveux par rapport aux polymères utilisés seuls (Compositions C et D).

## Revendications

1. Composition cosmétique ou dermatologique destinée au traitement des matières kératiniques, comprenant dans un milieu cosmétiquement ou dermatologiquement acceptable, au moins un polymère siliconé greffé, à squelette polysiloxanique greffé par des monomères organiques non-siliconés et au moins un polymère anionique dans un rapport en poids polymère anionique / polymère siliconé greffé compris entre 0,25 et 15, **caractérisée par le fait que** :
(i) le polymère anionique est un polymère fixant, et
(ii) le polymère siliconé greffé est choisi parmi les polymères siliconés comportant dans leur structure le motif de formule (I) suivant: 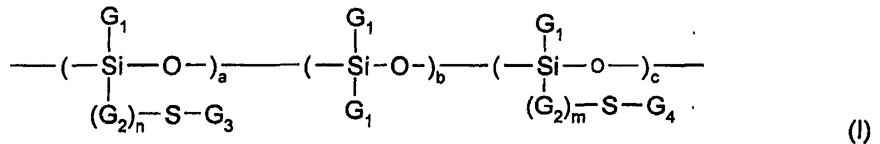
dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀;
G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ;
a est un nombre entier allant de 0 et 50 ; b est un nombre entier étant compris entre 10 et 350, c est un nombre entier allant de 0 et 50 ; sous réserve que l'un des paramètres a et c soit différent de 0.

2. Composition selon la revendication 1, **caractérisée par le fait que** le monomère organique anionique à insaturation éthylénique est choisi, seul ou sous forme de mélange de monomères, parmi les acides carboxyliques insaturés, linéaires ou ramifiés.

3. Composition selon la revendication 2, **caractérisée par le fait que** le monomère organique anionique à insaturation éthylénique est choisi, seul ou sous forme de mélange de monomères, parmi l'acide acrylique, l'acide méthacrylique, l'acide maléique, l'anhydride maléique, l'acide itaconique, l'acide fumarique et l'acide crotonique.

4. Composition selon la revendication 1, **caractérisée par le fait que** le monomère organique hydrophobe à insaturation éthylénique est choisi, seul ou en mélange de monomères, parmi les esters d'acide acrylique et d'alcanol et/ou les esters d'acide méthacrylique et d'alcanol, de préférence l'alcanol étant en C₁-C₁₈.

5. Composition selon la revendication 4, **caractérisée par le fait que** le monomère organique hydrophobe à insaturation éthylénique est choisi, seul ou en mélange de monomères dans le groupe constitué par le (méth)acrylate d'isooctyle, le 2-éthylhexyl(méth)acrylate, le (méth)acrylate de lauryle, le (méth) acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle.

6. Composition selon l'une quelconque des revendications 1 à 5, **caractérisée par le fait que** le polymère siliconé greffé comprend sur la chaîne silicone principale, au moins un groupement organique à caractère anionique obtenu par l'(homo) polymérisation radicalaire d'au moins un monomère anionique de type acide carboxylique insaturé, partiellement ou totalement neutralisé sous la forme d'un sel.

7. Composition selon l'une quelconque des revendications précédentes, **caractérisée par le fait que** le motif de formule (I) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle en C₁-C₁₀.

8. Composition selon l'une quelconque des revendications précédentes, **caractérisée par le fait que** le motif de formule (I) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobutyle ou de méthyle.

9. Composition selon l'une quelconque des revendications précédentes, **caractérisée par le fait que** la masse moléculaire en nombre du polymère siliconé greffé varie de 10 000 à 1 000 000 environ, et encore plus préférentiellement de 10 000 à 100000 environ.

10. Composition selon l'une quelconque des revendications précédentes, **caractérisée par le fait que** le polymère siliconé greffé est utilisé en une quantité allant de 0,01 à 15% en poids par rapport au poids total de la composition et de préférence de 0,1 à 10% en poids.

11. Composition selon l'une des revendications précédentes, **caractérisée par le fait que** le polymère anionique est choisi parmi :
- les polymères comportant des motifs carboxyliques dérivant de monomères mono ou diacides carboxyliques insaturés de formule: 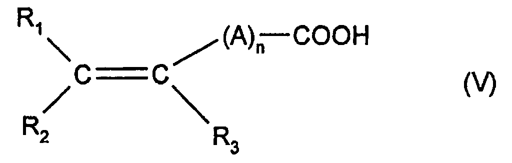
dans laquelle n est un nombre entier de 0 à 10, A désigne un groupement méthylène, éventuellement relié à l'atome de carbone du groupement insaturé
ou au groupement méthylène voisin lorsque n est supérieur à 1 par l'intermédiaire d'un hétéroatome tel que oxygène ou soufre, R₁ désigne un atome d'hydrogène, un groupement phényle ou benzyle, R₂ désigne un atome d'hydrogène, un groupement alkyle inférieur en C1 à C4 ou carboxyle, R₃ désigne un atome d'hydrogène, un groupement alkyle inférieur en C1 à C4, un groupement -CH₂-COOH, phényle ou benzyle;
- les polymères comprenant des motifs dérivant d'acide sulfonique tels que des motifs vinylsulfonique, styrènesulfonique, acrylamido alkylsulfonique.

12. Composition selon la revendication 11, **caractérisée en ce que** le polymère anionique est choisi parmi :
A) les homo- ou copolymères d'acide acrylique ou méthacrylique ou leurs sels, les copolymères d'acide acrylique et d'acrylamide et leurs sels, les sels de sodium d'acides polyhydroxycarboxyliques ;
B) les copolymères des acides acrylique ou méthacryliques avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés ; les copolymères de ce type comportant dans leur chaîne un motif acrylamide éventuellement N-alkylé et/ou hydroxyalkylé, les copolymères d'acide acrylique et de méthacrylate d'alkyle en C₁-C₄;
C) les copolymères dérivés d'acide crotonique tels que ceux comportant dans leur chaîne des motif acétate ou propionate de vinyle et éventuellement. d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ;
D) les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acrylique ou méthacrylique ou la vinylpyrrolidone dans leur chaîne, les fonctions anhydrides sont monoestérifiées ou monoamidifiées ;
E) les polyacrylamides comportant des groupements carboxylates.

13. Composition selon la revendication 12, **caractérisée en ce que** le polymère anionique est choisi parmi :
- les copolymères d'acide acrylique tels que le terpolymère acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- le copolymère d'acide méthacrylique et d'acrylate d'éthyle;
- le copolymère acétate de vinyle/acide crotonique;
- le terpolymère acétate de vinyle/acide crotonique/polyéthylèneglycol.

14. Composition selon l'une quelconque des revendications précédentes, **caractérisée par** le fait le rapport en poids polymère anionique / polymère siliconé greffé est compris entre 0,3 et 8.

15. Composition selon l'une quelconque des revendications précédentes, **caractérisée par le fait que** le polymère anionique est utilisé en une quantité allant de 0, 1 à 20% en poids par rapport au poids total de la composition et de préférence de 0,2 à 15% en poids et encore plus préférentiellement de 0,5 à 10 % en poids.

16. Composition selon l'une quelconque des revendications précédentes, **caractérisée par le fait qu'**elle contient en plus au moins un additif choisi dans le groupe constitué par les épaississants, lés esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

17. Composition selon l'une quelconque des revendications précédentes, **caractérisée par le fait que** le milieu cosmétiquement ou dermatologiquement acceptable est constitué par de l'eau ou un mélange d'eau et d'au moins un solvant cosmétiquement acceptable.

18. Composition selon la revendication 17, **caractérisée par le fait que** les solvants cosmétiquement acceptables sont choisis dans le groupe constitué par les monoalcools, les polyalcools, les éthers de glycol, les esters d'acides gras et leurs mélanges.

19. Composition selon l'une quelconque des revendications précédentes, **caractérisée par le fait que** le polymère siliconé greffé est dissous dans le milieu cosmétiquement ou dermatologiquement acceptable ou utilisé sous forme de dispersion aqueuse de particules .

20. Composition selon l'une quelconque des revendications précédentes, **caractérisée par le fait que** les matières kératiniques sont des cheveux humains.

21. Composition selon l'une quelconque des revendications précédentes, **caractérisée par le fait qu'**elle se présente sous forme de gel, de lait, de crème, de lotion plus ou moins épaissie ou de mousse.

22. Composition selon l'une quelconque des revendications précédentes, **caractérisée par le fait qu'**elle est un produit de coiffage.

23. Composition selon l'une quelconque des revendications précédentes, **caractérisée par le fait qu'**elle est un produit capillaire choisi dans le groupe constitué par des shampooings ; des produits capillaires à rincer ou non, à appliquer avant ou après un shampooing, une coloration, une décoloration, une permanente ou un défrisage.

24. Composition selon l'une quelconque des revendications précédentes, **caractérisée par le fait qu'**elle est conditionnée sous forme de vaporisateur, de flacon pompe ou bien dans un récipient aérosol en vue d'obtenir un spray, une laque ou une mousse.

25. Procédé non-thérapeutique de traitement des matières kératiniques en particulier des cheveux humains, **caractérisé par le fait qu'**il consiste à appliquer sur lesdites matières une composition telle que définie selon l'une quelconque des revendications 1 à 24 puis à effectuer éventuellement un rinçage à l'eau.

## Patentansprüche

1. Kosmetische oder dermatologische Zusammensetzung, die zur Behandlung von Keratinsubstanzen vorgesehen ist und die in einem kosmetisch oder dermatologisch akzeptablen Medium mindestens ein gepfropftes Siliconpolymer mit Polysiloxangerüst, auf das nicht siliconhaltige, organische Monomere gepfropft sind, und mindestens ein anionisches Polymer in einem Gewichtsverhältnis anionisches Polymer/gepfropftes Siliconpolymer im Bereich von 0,25 bis 15 enthält, **dadurch gekennzeichnet, daß**
(i) das anionische Polymer ein fixierendes Polymer ist, und
(ii) das gepfropfte Siliconpolymer unter den Siliconpolymeren ausgewählt ist, die in ihrer Struktur die Einheit der folgenden Formel (I) enthalten: 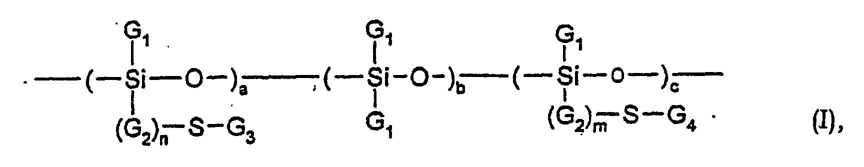
worin die Gruppen G₁, die identisch oder voneinander verschieden sind, Wasserstoff, C₁₋₁₀-Alkyl oder Phenyl; die Gruppen G₂, die identisch oder voneinander verschieden sind, C₁₋₁₀-Alkylen; G₃ eine Polymergruppe, die aus der (Homo)polymerisation mindestens eines anionischen Monomers mit ethylenischer Doppelbindung resultiert; G₄ eine Polymergruppe, die aus der (Homo)polymerisation mindestens eines hydrophoben Monomers mit ethylenischer Doppelbindung resultiert; m und n 0 oder 1; a 0 oder eine ganze Zahl im Bereich von 1 bis 50; b eine ganze Zahl im Bereich von 10 bis 350 und c 0 oder eine ganze Zahl von 1 bis 50 bedeuten, mit der Maßgabe, daß einer der Parameter a oder c von 0 verschieden ist.

2. Zusammensetzung nach Anspruch 1, **dadurch gekennzeichnet, daß** das anionische organische Monomer mit ethylenischer Doppelbindung einzeln oder in Form von Monomerengemischen unter den geradkettigen oder verzweigten, ungesättigten Carbonsäuren ausgewählt ist.

3. Zusammensetzung nach Anspruch 2, **dadurch gekennzeichnet, daß** das anionische organische Monomer mit ethylenischer Doppelbindung einzeln oder in Form von Monomerengemischen unter Acrylsäure, Methacrylsäure, Maleinsäure, Maleinsäureanhydrid, Itaconsäure, Fumarsäure und Crotonsäure ausgewählt ist.

4. Zusammensetzung nach Anspruch 1, **dadurch gekennzeichnet, daß** das hydrophobe organische Monomer mit ethylenischer Doppelbindung einzeln oder in Form von Monomerengemischen unter den Estern von Acrylsäure und einem Alkanol und/oder den Estern von Methacrylsäure und einem Alkanol ausgewählt sind, wobei das Alkanol vorzugsweise 1 bis 18 Kohlenstoffatome aufweist.

5. Zusammensetzung nach Anspruch 4, **dadurch gekennzeichnet, daß** das hydrophobe, organische Monomer mit ethylenischer Doppelbindung einzeln oder in Form von Monomerengemischen unter Isooctyl(meth)acrylat, 2-Ethylhexyl(meth)acrylat, Lauryl(meth)acrylat, Isopentyl(meth)acrylat, n-Butyl(meth)acrylat, Isobutyl(meth)acrylat, Methyl(meth)acrylat, t-Butyl(meth)acrylat, Tridecyl(meth)acrylat und Stearyl(meth)acrylat ausgewählt ist.

6. Zusammensetzung nach einem der Ansprüche 1 bis 5, **dadurch gekennzeichnet, daß** das gepfropfte Siliconpolymer in der Siliconhauptkette mindestens eine organische Gruppe mit anionischen Eigenschaften aufweist, die durch radikalische (Homo)polymerisation mindestens eines anionischen Monomers vom Typ einer ungesättigten, zum Teil oder vollständig in Form eines Salzes neutralisierten Carbonsäure hergestellt wird.

7. Zusammensetzung nach einem der vorhergehenden Ansprüche, **dadurch gekennzeichnet, daß** die Einheit der Formel I mindestens eine der folgenden Eigenschaften aufweist:
- die Gruppen G₁ bedeuten eine C₁₋₁₀₋Alkylgruppe;
- n ist nicht 0 und die Gruppen G₂ bedeuten eine zweiwertige Gruppe mit 1 bis 3 Kohlenstoffatomen;
- G₃ bedeutet eine Polymergruppe, die bei der (Homo)polymerisation mindestens eines Monomers vom Typ einer Carbonsäure mit ethylenischer Doppelbindung gebildet wird; und
- G₄ bedeutet eine Polymergruppe, die bei der (Homo)polymerisation mindestens eines Monomers vom Typ C₁₋₁₀-Alkyl(meth)acrylat entsteht.

8. Zusammensetzung nach einem der vorhergehenden Ansprüche, **dadurch gekennzeichnet, daß** die Einheit der Formel (I) alle folgenden Eigenschaften aufweist:
- die Gruppen G₁ bedeuten Methyl;
- n ist nicht 0 und die Gruppen G₂ bedeuten Propylen;
- G₃ bedeutet eine Polymergruppe, die bei der (Homo)polymerisation von zumindest Acrylsäure und/oder Methacrylsäure entsteht;
- G₄ bedeutet eine Polymergruppe, die bei der (Homo)polymerisation mindestens eines Monomers vom Typ Isobutyl(meth)acrylat oder Methyl(meth)acrylat entsteht.

9. Zusammensetzung nach einem der vorhergehenden Ansprüche, **dadurch gekennzeichnet, daß** das Zahlenmittel des Molekulargewichts des gepfropften Siliconpolymers im Bereich von etwa 10 000 bis 1 000 000 und noch bevorzugter von etwa 10 000 bis 100 000 liegt.

10. Zusammensetzung nach einem der vorhergehenden Ansprüche, **dadurch gekennzeichnet, daß** das gepfropfte Siliconpolymer in einer Menge von 0,01 bis 15 Gew.-%, bezogen auf das Gesamtgewicht der Zusammensetzung, und vorzugsweise 0,1 bis 10 Gew.-% verwendet wird.

11. Zusammensetzung nach einem der vorhergehenden Ansprüche, **dadurch gekennzeichnet, daß** das anionische Polymer ausgewählt ist unter:
- den Polymeren, die Carboxyeinheiten aufweisen, die von ungesättigten Mono- oder Dicarbonsäuremonomeren der Formel: 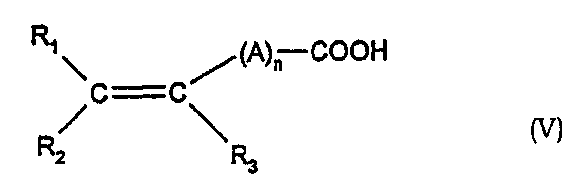 abgeleitet sind, wobei n 0 oder eine ganze Zahl von 1 bis 10 bedeutet, A eine Methylengruppe ist, die gegebenenfalls an das Kohlenstoffatom der ungesättigten Gruppe oder, wenn n größer 1 ist, an die benachbarte Methylengruppe über ein Heteroatom, wie Sauerstoff oder Schwefel, gebunden ist, R₁ Wasserstoff, Phenyl oder Benzyl bedeutet, R₂ Wasserstoff, eine niedere C₁₋₄-Alkylgruppe oder Carboxy ist und R₃ Wasserstoff, eine niedere C₁₋₄-Alkylgruppe, -CH₂-COOH, Phenyl oder Benzyl bedeutet; und
- den Polymeren, die Einheiten enthalten, die von Sulfonsäuren abgeleitet sind, wie Vinylsulfonsäureeinheiten, Styrolsulfonsäureeinheiten und Acrylamidoalkylsulfonsäureeinheiten.

12. Zusammensetzung nach Anspruch 11, **dadurch gekennzeichnet, daß** das anionische Polymer ausgewählt ist unter:
A) den Homo- oder Copolymeren von Acrylsäure oder Methacrylsäure oder deren Salzen, Copolymeren von Acrylsäure und Acrylamid und deren Salzen und Natriumsalzen von Polyhydroxycarbonsäuren;
B) den Copolymeren von Acrylsäure oder Methacrylsäure und einem monoethylenisch ungesättigten Monomer, wie Ethylen, Styrol, Vinylestern, Acrylsäure- oder Methacrylsäureestern, die gegebenenfalls mit einem Polyalkylenglykol, wie Polyethylenglykol, gepfropft sind und gegebenenfalls vernetzt sind; den Copolymeren dieses Typs, die in der Kette eine gegebenenfalls N-alkylierte und/oder hydroxyalkylierte Acrylamideinheit enthalten, und den Copolymeren von Acrylsäure und C₁₋₄-Alkylmethacrylat;
C) den Copolymeren, die von Crotonsäure abgeleitet sind, wie den Copolymeren, die in ihrer Kette Vinylacetat- oder Vinylpropionateinheiten und gegebenenfalls weitere Monomere enthalten, wie Allyl- oder Methallylester, Vinylether oder Vinylester einer gesättigten, geradkettigen oder verzweigten Carbonsäure mit langer Kohlenwasserstoffkette, beispielsweise Verbindungen, die mindestens 5 Kohlenstoffatome enthalten, wobei diese Polymere gegebenenfalls gepfropft und vernetzt vorliegen können;
D) den Copolymeren von Maleinsäure, Fumarsäure, Itaconsäure oder deren Anhydriden und Vinylestern, Vinylethern, Vinylhalogeniden, Phenylvinylderivaten und Acrylsäure und ihren Estern; Copolymeren von Maleinsäure, Citraconsäure, Itaconsäure oder deren Anhydriden und Allylestern oder Methallylestern, die gegebenenfalls eine oder mehrere Gruppen Acrylamid, Methacrylamid, α-Olefin, Acrylester oder Methacrylester, Acrylsäure oder Methacrylsäure oder Vinylpyrrolidon in ihrer Kette enthalten, wobei die Anhydridfunktionen dieser Copolymere einfach verestert oder amidiert sind; und
(E) den Polyacrylamiden, die Carboxygruppen aufweisen.

13. Zusammensetzung nach Anspruch 12, **dadurch gekennzeichnet, daß** das anionische Polymer ausgewählt ist unter:
- den Acrylsäurecopolymeren, beispielsweise dem Acrylsäure/Ethylacrylat/N-t-Butylacrylamid-Terpolymer;
- den Copolymeren, die von Crotonsäure abgeleitet sind, beispielsweise den Vinylacetat/Vinyl-t-Butylbenzoat/Crotonsäure-Terpolymeren und den Crotonsäure/Vinylacetat/Vinylneododecanoat-Terpolymeren;
- den Polymeren, die von Maleinsäure, Fumarsäure, Itaconsäure oder deren Anhydriden und Vinylestern, Vinylethern, Vinylhalogeniden, Phenylvinylderivaten und Acrylsäure und ihren Estern abgeleitet sind, beispielsweise den Copolymeren von Methylvinylether und einfach verestertem Maleinsäureanhydrid;
- den Copolymeren von Methacrylsäure und Methylmethacrylat;
- dem Copolymer von Methacrylsäure und Ethylacrylat;
- dem Copolymer von Vinylacetat und Crotonsäure; und
- den Vinylacetat/Crotonsäure/ Polyethylenglykol-Terpolymeren.

14. Zusammensetzung nach einem der vorhergehenden Ansprüche, **dadurch gekennzeichnet, daß** das Verhältnis anionisches Polymer/gepfropftes Siliconpolymer im Bereich von 0,3 bis 8 liegt.

15. Zusammensetzung nach einem der vorhergehenden Ansprüche, **dadurch gekennzeichnet, daß** das anionische Polymer in einer Menge von 0,1 bis 20 Gew.-%, bezogen auf das Gesamtgewicht der Zusammensetzung, vorzugsweise 0,2 bis 15 Gew.-% und noch bevorzugter 0,5 bis 10 Gew.-% verwendet wird.

16. Zusammensetzung nach einem der vorhergehenden Ansprüche, **dadurch gekennzeichnet, daß** sie ferner mindestens einen Zusatzstoff enthält, der unter den Verdickungsmitteln, Fettsäureestern, Estern von Fettsäuren und Glycerin, Siliconen, grenzflächenaktiven Stoffen, Parfums, Konservierungsmitteln, Sonnenschutzfiltern, Proteinen, Vitaminen, Polymeren, pflanzlichen Ölen, tierischen Ölen, Mineralölen oder synthetischen Ölen oder beliebigen herkömmlich in der Kosmetik verwendeten Zusatzstoffen ausgewählt ist.

17. Zusammensetzung nach einem der vorhergehenden Ansprüche, **dadurch gekennzeichnet, daß** das kosmetisch oder dermatologisch akzeptable Medium aus Wasser oder einem Gemisch von Wasser und mindestens einem kosmetisch akzeptablen Lösungsmittel besteht.

18. Zusammensetzung nach Anspruch 17, **dadurch gekennzeichnet, daß** die kosmetisch akzeptablen Lösungsmittel unter den Monoalkoholen, Polyalkoholen, Glykolethern, Fettsäureestern und deren Gemischen ausgewählt sind.

19. Zusammensetzung nach einem der vorhergehenden Ansprüche, **dadurch gekennzeichnet, daß** das gepfropfte Siliconpolymer in dem kosmetisch oder dermatologisch akzeptablen Medium gelöst ist oder in Form einer wäßrigen Dispersion von Partikeln verwendet wird.

20. Zusammensetzung nach einem der vorhergehenden Ansprüche, **dadurch gekennzeichnet, daß** es sich bei den Keratinsubstanzen um menschliches Haar handelt.

21. Zusammensetzung nach einem der vorhergehenden Ansprüche, **dadurch gekennzeichnet, daß** sie als Gel, Milch, Creme, mehr oder weniger dickflüssige Lotion oder Schaum vorliegt.

22. Zusammensetzung nach einem der vorhergehenden Ansprüche, **dadurch gekennzeichnet, daß** es sich um ein Produkt zum Frisieren handelt.

23. Zusammensetzung nach einem der vorhergehenden Ansprüche, **dadurch gekennzeichnet, daß** es sich um ein Produkt für das Haar handelt, das unter den Haarwaschmitteln und Produkten für das. Haar, die ausgespült oder nicht ausgespült werden und vor oder nach einer Haarwäsche, Färbung, Entfärbung, Dauerwelle oder Entkräuselung aufgetragen werden, ausgewählt ist.

24. Zusammensetzung nach einem der vorhergehenden Ansprüche, **dadurch gekennzeichnet, daß** sie zur Bildung eines Sprays, eines Lacks oder eines Schaums in einem Zerstäuber, Pumpflakon oder Aersolbehälter konfektioniert ist.

25. Nicht therapeutisches Verfahren zur Behandlung von Keratinsubstanzen und insbesondere menschlichem Haar, **dadurch gekennzeichnet, daß** es darin besteht, auf die Keratinsubstanzen eine Zusammensetzung nach einem der Ansprüche 1 bis 24 aufzutragen und gegebenenfalls mit Wasser zu spülen.

## Claims

1. Cosmetic or dermatological composition intended for treating keratin materials, comprising, in a cosmetically or dermatologically acceptable medium, at least one grafted silicone polymer with a polysiloxane skeleton grafted with non-silicone organic monomers and at least one anionic polymer in an anionic polymer/grafted silicone polymer weight ratio of between 0.25 and 15, **characterized in that**:
(i) the anionic polymer is a fixing polymer, and
(ii) the grafted silicone polymer is chosen from silicone polymers comprising in their structure the unit of formula (I) below: 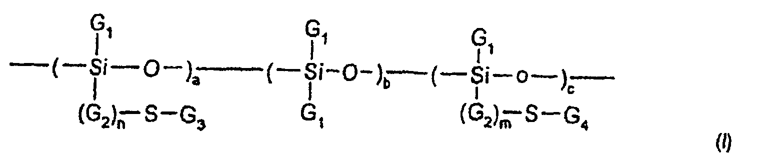 in which the radicals G₁, which may be identical or different, represent hydrogen or a C₁-C₁₀ alkyl radical or a phenyl radical; the radicals G₂, which may be identical or different, represent a C₁-C₁₀ alkylene group; G₃ represents a polymer residue resulting from the (homo)polymerization of at least one ethylenically unsaturated anionic monomer; G₄ represents a polymer residue resulting from the (homo)polymerization of at least one ethylenically unsaturated hydrophobic monomer; m and n are equal to 0 or 1; a is an integer ranging from 0 to 50; b is an integer between 10 and 350, c is an integer ranging from 0 to 50; with the proviso that one of the parameters a and c is other than 0.

2. Composition according to Claim 1, **characterized in that** the ethylenically unsaturated anionic organic monomer is chosen, alone or in the form of a monomer mixture, from linear or branched unsaturated carboxylic acids.

3. Composition according to Claim 2, **characterized in that** the ethylenically unsaturated anionic organic monomer is chosen, alone or in the form of a monomer mixture, from acrylic acid, methacrylic acid, maleic acid, maleic anhydride, itaconic acid, fumaric acid and crotonic acid.

4. Composition according to Claim 1, **characterized in that** the ethylenically unsaturated hydrophobic organic monomer is chosen, alone or as a monomer mixture, from acrylic acid esters of an alkanol and/or methacrylic acid esters of an alkanol, the alkanol preferably being C₁-C₁₈.

5. Composition according to Claim 4, **characterized in that** the ethylenically unsaturated hydrophobic organic monomer is chosen, alone or as a monomer mixture, from the group consisting of isooctyl (meth)acrylate, 2-ethylhexyl (meth)acrylate, lauryl (meth)acrylate, isopentyl (meth)acrylate, n-butyl (meth)acrylate, isobutyl (meth)acrylate, methyl (meth)acrylate, tert-butyl (meth)acrylate, tridecyl (meth)acrylate and stearyl (meth)acrylate.

6. Composition according to any one of Claims 1 to 5, **characterized in that** the grafted silicone polymer comprises on the main silicone chain at least one organic group of anionic nature obtained by the free-radical (homo)polymerization of at least one anionic monomer of unsaturated carboxylic acid type, partially or totally neutralized in the form of a salt.

7. Composition according to any one of the preceding claims, **characterized in that** the unit of formula (I) has at least one of the following characteristics:
- the radicals G₁ denote a C₁-C₁₀ alkyl radical;
- n is non-zero, and the radicals G₂ represent a divalent C₁-C₃ radical;
- G₃ represents a polymer radical resulting from the (homo)polymerization of at least one monomer of the ethylenically unsaturated carboxylic acid type;
- G₄ represents a polymer radical resulting from the (homo)polymerization of at least one monomer of the C₁-C₁₀ alkyl (meth)acrylate type.

8. Composition according to any one of the preceding claims, **characterized in that** the unit of formula (I) simultaneously has the following characteristics:
- the radicals G₁ denote a methyl radical;
- n is non-zero, and the radicals G₂ represent a propylene radical;
- G₃ represents a polymer radical resulting from the (homo)polymerization of at least acrylic acid and/or methacrylic acid;
- G₄ represents a polymer radical resulting from the (homo)polymerization of at least one monomer of the isobutyl or methyl (meth)acrylate type.

9. Composition according to any one of the preceding claims, **characterized in that** the number-average molecular mass of the grafted silicone polymer ranges from 10 000 to 1 000 000 approximately and even more preferably from 10 000 to 100 000 approximately.

10. Composition according to any one of the preceding claims, **characterized in that** the grafted silicone polymer is used in an amount ranging from 0.01% to 15% by weight relative to the total weight of the composition, and preferably from 0.1% to 10% by weight.

11. Composition according to one of the preceding claims, **characterized in that** the anionic polymer is chosen from:
- polymers comprising carboxylic units derived from unsaturated monocarboxylic or dicarboxylic acid monomers of formula: 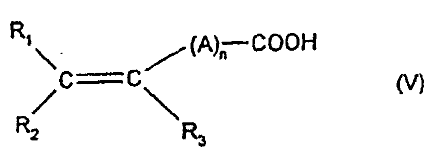 in which n is an integer from 0 to 10, A denotes a methylene group, optionally linked to the carbon atom of the unsaturated group or to the neighbouring methylene group when n is greater than 1 via a hetero atom such as oxygen or sulphur, R₁ denotes a hydrogen atom or a phenyl or benzyl group, R₂ denotes a hydrogen atom or a C1 to C4 lower alkyl or carboxyl group, R₃ denotes a hydrogen atom, a C1 to C4 lower alkyl group or a -CH₂-COOH, phenyl or benzyl group,
- polymers comprising units derived from sulphonic acid, such as vinylsulphonic, styrenesulphonic or acrylamidoalkylsulphonic units.

12. Composition according to Claim 11, **characterized in that** the anionic polymer is chosen from:
A) homopolymers or copolymers of acrylic or methacrylic acid or salts thereof, copolymers of acrylic acid and of acrylamide and salts thereof, sodium salts of polyhydroxycarboxylic acids;
B) copolymers of acrylic or methacrylic acids with a monoethylenic monomer such as ethylene, styrene, vinyl esters, acrylic or methacrylic acid esters, optionally grafted onto a polyalkylene glycol such as polyethylene glycol and optionally crosslinked; the copolymers of this type comprising in their chain an optionally N-alkylated and/or hydroxyalkylated acrylamide unit, copolymers of acrylic acid and of C₁-C₄ alkyl methacrylate;
C) copolymers derived from crotonic acid, such as those comprising in their chain vinyl acetate or propionate units and optionally other monomers such as allylic or methallylic esters, vinyl ether or vinyl ester of a saturated, linear or branched carboxylic acid containing a long hydrocarbon-based chain, such as those comprising at least 5 carbon atoms, these polymers possibly being grafted and crosslinked;
D) polymers derived from maleic, fumaric or itaconic acids or anhydrides with vinyl esters, with vinyl ethers, with vinyl halides or with phenylvinyl derivatives, and acrylic acid and its esters; copolymers of maleic, citraconic or itaconic anhydrides and of an allylic or methallylic ester optionally comprising an acrylamide or methacrylamide group, an α-olefin, acrylic or methacrylic esters, acrylic or methacrylic acids or vinylpyrrolidone in their chain, the anhydride functions being monoesterified or monoamidated;
E) polyacrylamides comprising carboxylate groups.

13. Composition according to Claim 12, **characterized in that** the anionic polymer is chosen from:
- copolymers of acrylic acid, such as the acrylic acid/ethyl acrylate/N-tert-butylacrylamide terpolymer;
- copolymers derived from crotonic acid, such as vinyl acetate/vinyl tert-butyl benzoate/crotonic acid terpolymers and crotonic acid/vinyl acetate/vinyl neododecanoate terpolymers;
- polymers derived from maleic, fumaric or itaconic acids or anhydrides with vinyl esters, with vinyl ethers, with vinyl halides or with phenylvinyl derivatives, and acrylic acid and its esters, such as methyl vinyl ether/monoesterified maleic anhydride copolymers;
- copolymers of methacrylic acid and of methyl methacrylate;
- the copolymer of methacrylic acid and of ethyl acrylate;
- the vinyl acetate/crotonic acid copolymer;
- the vinyl acetate/crotonic acid/polyethylene glycol terpolymer.

14. Composition according to any one of the preceding claims, **characterized in that** the anionic polymer/grafted silicone polymer weight ratio is between 0.3 and 8.

15. Composition according to any one of the preceding claims, **characterized in that** the anionic polymer is used in an amount ranging from 0.1% to 20% by weight relative to the total weight of the composition, preferably from 0.2% to 15% by weight and even more preferably from 0.5% to 10% by weight.

16. Composition according to any one of the preceding claims, **characterized in that** it also contains at least one additive chosen from the group consisting of thickeners, fatty acid esters, fatty acid esters of glycerol, silicones, surfactants, fragrances, preserving agents, sunscreens, proteins, vitamins, polymers, plant, animal, mineral or synthetic oils and any other additive conventionally used in cosmetics.

17. Composition according to any one of the preceding claims, **characterized in that** the cosmetically or dermatologically acceptable medium consists of water or a mixture of water and at least one cosmetically acceptable solvent.

18. Composition according to Claim 17, **characterized in that** the cosmetically acceptable solvents are chosen from the group consisting of monoalcohols, polyalcohols, glycol ethers and fatty acid esters, and mixtures thereof.

19. Composition according to any one of the preceding claims, **characterized in that** the grafted silicone polymer is dissolved in the cosmetically or dermatologically acceptable medium or used in the form of an aqueous dispersion of particles.

20. Composition according to any one of the preceding claims, **characterized in that** the keratin materials are human hair.

21. Composition according to any one of the preceding claims, **characterized in that** it is in the form of a gel, a milk, a cream, a more or less thickened lotion or a mousse.

22. Composition according to any one of the preceding claims, **characterized in that** it is a hair styling product.

23. Composition according to any one of the preceding claims, **characterized in that** it is a hair product chosen from the group consisting of shampoos; rinse-out or leave-in hair products, to be applied before or after shampooing, dyeing, bleaching, permanent-waving or straightening the hair.

24. Composition according to any one of the preceding claims, **characterized in that** it is packaged in the form of a vaporizer or a pump-dispenser bottle, or alternatively in an aerosol container in order to obtain a spray, a lacquer or a mousse.

25. Non-therapeutic process for treating keratin materials, in particular human hair, **characterized in that** it consists in applying to the said materials a composition as defined according to any one of Claims 1 to 24, and then in optionally rinsing the materials with water.
